(19)

Europäisches Patentamt

European Patent Office

Office européen des brevets

(11) **EP 4 521 337 A1**

(12) **EUROPEAN PATENT APPLICATION**

(43) Date of publication:
**12.03.2025 Bulletin 2025/11**

(21) Application number: **24198548.0**

(22) Date of filing: **05.09.2024**

(51) International Patent Classification (IPC):
**G06T 5/40** *(2006.01)* **G06T 5/92** *(2024.01)*

(52) Cooperative Patent Classification (CPC):
**G06T 5/92; G06T 5/40;** G06T 2207/10056;
G06T 2207/10068

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.09.2023 JP 2023144933**

(71) Applicant: **Evident Corporation
Kamiina-gun, Nagano 399-0495 (JP)**

(72) Inventor: **NAKATSUKA, Masayuki
Tatsuno-machi, Kamiina-gun, Nagano, 3990495
(JP)**

(74) Representative: **Schicker, Silvia
Wuesthoff & Wuesthoff
Patentanwälte und Rechtsanwalt PartG mbB
Schweigerstraße 2
81541 München (DE)**

(54) **MICROSCOPE SYSTEM, METHOD OF IMAGE PROCESSING, AND PROGRAM**

(57) A microscope system includes: a microscope that forms an optical observation image; an image capturing device that captures the optical observation image and generates an observation image; a histogram generation unit that generates a histogram plotting pixel values of every color component in the observation image; an adjustment value determination unit that determines a black balance adjustment value based on the histogram plotting pixel values of every color component; an adjustment unit that subtracts the black balance adjustment value from pixel values of every color component in each pixel of the observation image to generate an adjusted observation image; and an output device that outputs the adjusted observation image.

EP 4 521 337 A1

**Description**

BACKGROUND OF THE INVENTION

TECHNICAL FIELD

The present disclosure relates to a microscope system, a method of image processing, and a program.

DESCRIPTION OF THE RELATED ART

Fluorescence observation is known as one of observation methods using a microscope. In fluorescence observation, a specific site, or a specific protein or molecule, in a cell is visualized using a fluorescent dye or a fluorescent protein.

In fluorescence observation, autofluorescence of an optical member (for example, an objective lens) included in a microscope potentially causes fluorescence not only in a sample but also in a background of an observation image, which results in reduction of contrast between the sample and the background. In order to prevent such reduction of contrast, a technique of adjusting black balance is employed. In adjusting black balance, brightness of the background (brightness due to autofluorescence of the optical member) is subtracted from the observation image to clarify a difference in brightness between the sample and the background, thereby obtaining a high-contrast observation image.

JP 2010-098719 A discloses a microscope system that adjusts black balance. The microscope system of JP 2010-098719 A generates a brightness distribution of an observation image, detects the brightest displacement point as a high brightness displacement point and the darkest displacement point as a low brightness displacement point among displacement points in the brightness distribution, calculates a difference between a brightness value of the high brightness displacement point and a brightness value of the low brightness displacement point, determines the brightness value of the low brightness displacement point as an adjustment value according to the difference, performs adjustment by subtracting the adjustment value from a brightness value of each pixel constituting the observation image based on the determination result, and outputs (for example, displays) the adjusted observation image.

SUMMARY OF THE INVENTION

In the microscope system disclosed in JP 2010-098719 A, the adjustment value (black balance adjustment value) is determined based on the brightness distribution (histogram of brightness) of the observation image.

However, in the microscope system of JP 2010-098719 A, for example, in a case where the observation image is a color image (each pixel has R, G, and B values), adjusting black balance by calculating brightness information based on color information causes the following problem.

Brightness information (Y value) of each pixel of the observation image is obtained from, for example, color information (R, G, and B values) of each pixel by the following Formula (1).

$$Y = 0.299*R + 0.587*G + 0.114*B \quad (1)$$

For example, assume that an observation target is fluorescence of the single blue (B) color. The red component (R value) and the green component (G value) in each pixel of the observation image are close to 0, and the brightness information (Y value) of each pixel obtained by Formula (1) is substantially multiplied by the smallest weighting coefficient (0.114) with respect to the blue component (B value) of each pixel. Accordingly, an adjustment value determined based on a histogram of brightness becomes small. If black balance is adjusted using such a small adjustment value, brightness of a background (brightness due to autofluorescence of an optical member) cannot be sufficiently subtracted from the observation image, which makes it difficult to remedy the reduction of contrast between a sample and the background of the observation image.

An object of one aspect of the present invention is to provide a technique for preferably adjusting black balance without the influence of color components in an observation image.

A microscope system according to an aspect of the present invention includes: a microscope that forms an optical observation image; an image capturing device that captures the optical observation image and generates an observation image; a histogram generation unit that generates a histogram plotting pixel values of every color component in the observation image; an adjustment value determination unit that determines a black balance adjustment value based on the histogram plotting pixel values of every color component; an adjustment unit that subtracts the black balance adjustment value from pixel values of every color component in each pixel of the observation image to generate an adjusted observation image; and an output device that outputs the adjusted observation image.

A method of image processing according to an aspect of the present invention is executed by a computer, the method involving: generating a histogram plotting pixel values of every color component in an observation image which is generated by an image capturing device capturing an optical observation image formed by a microscope; determining a black balance adjustment value based on the histogram plotting pixel values of every color component; and subtracting the black balance adjustment value from pixel values of every color component in each pixel of the observation image to generate an adjusted observation image.

A program according to an aspect of the present invention causes a computer to implement: generating a histogram plotting pixel values of every color component in an observation image which is generated by an image capturing device capturing an optical observation image formed by a microscope; determining a black balance adjustment value based on the histogram plotting pixel values of every color component; and subtracting the black balance adjustment value from pixel values of every color component in each pixel of the observation image to generate an adjusted observation image.

According to the above aspects, black balance can be preferably adjusted without the influence of color components in an observation image.

BRIEF DESCRIPTION OF THE DRAWINGS

FIG. 1 is a view illustrating a configuration of a microscope system according to an embodiment;
FIG. 2 is a view illustrating an example of part of operation performed by an adjustment value determination unit;
FIG. 3 is a flowchart illustrating image processing performed by an image processing device;
FIG. 4 is a view illustrating a configuration of the microscope system according to a first modification;
FIG. 5 is a view illustrating a configuration of the microscope system according to a second modification;
FIG. 6 is a view illustrating a configuration of the microscope system according to a third modification; and
FIG. 7 is a view illustrating a hardware configuration of a computer.

DETAILED DESCRIPTION

Hereinafter, embodiments of the present invention will be described with reference to the drawings.

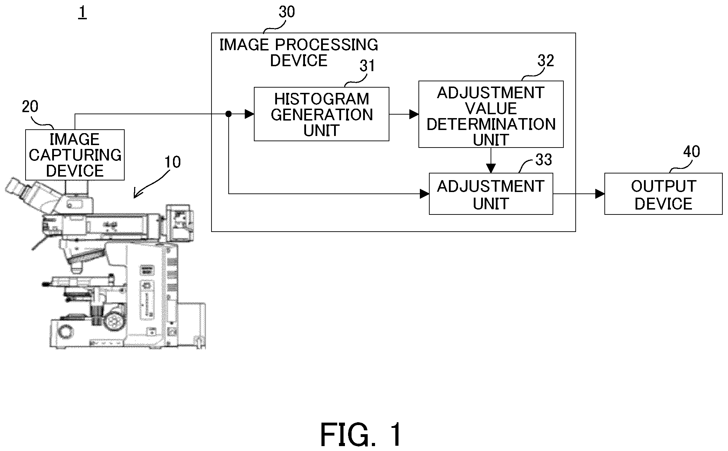

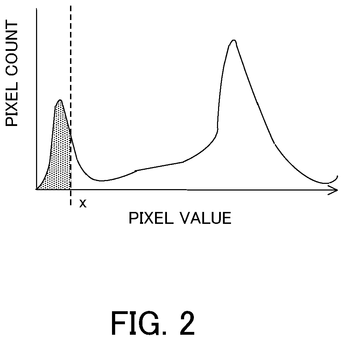

FIG. 1 is a view illustrating a configuration of a microscope system according to an embodiment. FIG. 2 is a view illustrating an example of part of operation performed by an adjustment value determination unit.

A microscope system 1 illustrated in FIG. 1 includes a microscope 10, an image capturing device 20, an image processing device 30, and an output device 40.

The microscope 10 forms an optical observation image of a sample. The microscope 10 enables fluorescence observation and forms, for example, an optical observation image at the time of fluorescence observation.

The image capturing device 20 is installed in the microscope 10 and captures the optical observation image formed by the microscope 10 to generate an observation image. The image capturing device 20 includes an image sensor such as a charge coupled device (CCD) and a complementary metal oxide semiconductor (CMOS), and the image sensor has, for example, a Bayer pattern color filter. The observation image generated by the image capturing device 20 may be a Bayer pattern image or a color image obtained by processing the Bayer pattern image. The processing involves conversion of the Bayer pattern image into the color image and involves, for example, de-mosaicing, color operation, gamma correction, edge enhancement, and noise reduction.

The image processing device 30 adjusts black balance of the observation image generated by the image capturing device 20. The image processing device 30 includes a histogram generation unit 31, an adjustment value determination unit 32, and an adjustment unit 33.

The histogram generation unit 31 generates a histogram plotting pixel values of every color component in the observation image generated by the image capturing device 20. For example, in a case where the observation image generated by the image capturing device 20 is an RGB color image (each pixel has RGB color components), the histogram generation unit 31 generates three histograms, that is, a histogram plotting pixel values of R component, a histogram plotting pixel values of G component, and a histogram plotting pixel values of B component. Alternatively, in a case where the observation image generated by the image capturing device 20 is a Bayer pattern image, the histogram generation unit 31 generates four histograms, that is, a histogram plotting pixel values of R component, a histogram plotting pixel values of Gr component, a histogram plotting pixel values of Gb component, and a histogram plotting pixel values of B component.

Hereinafter, data of a histogram generated by the histogram generation unit 31 is expressed as Hist[c][n]. The symbols c and n represent a color component and a signal level (pixel value), respectively. For example, in a case where the observation image is an RGB color image and a signal level of each color component is expressed by 8 bits, c becomes any of the color components R, G, and B, and n becomes any value of 0 to 255. In this case, for example, Hist[R][32] represents a pixel count where R component in the RGB color image has a signal level of 32.

The adjustment value determination unit 32 determines a black balance adjustment value based on the histogram plotting pixel values of every color component generated by the histogram generation unit 31. Specifically, the adjustment value determination unit 32 determines a black balance adjustment value in the following manner.

First, the adjustment value determination unit 32 determines, as a potential black balance adjustment value, a pixel value of every color component at which a cumulative pixel count from the darkest part of the histogram plotting pixel values of every color component reaches a threshold. Specifically, based on the data Hist[c][n] of the histogram plotting pixel values of every color component and a threshold Thr, a minimum x that satisfies the following Formula (2) is obtained for each color component.

$$Thr \leq \sum_{n=0}^{x} Hist[c][n] \quad (2)$$

The minimum x satisfying Formula (2) is a pixel value at which a cumulative pixel count from the darkest part of the histogram reaches the threshold Thr as illustrated in FIG. 2 (solid portion in FIG. 2), and each minimum x is determined as a potential black balance adjustment value. Hereinafter, x obtained for each color component using Formula (2) is expressed as x[c]. The symbol c represents a color component.

Next, the adjustment value determination unit 32 determines, as a black balance adjustment value, a maximum of potential black balance adjustment values determined for every color component. Specifically, a black balance adjustment value X is determined from x [c] of each color component by the following Formula (3) or the following Formula (4). Note that the following Formula (3) is used in a case where the observation image is an RGB color image, and the following formula (4) is used in a case where the observation image is a Bayer pattern image.

$$X = MAX(x[R], x[G], x[B]) \quad (3)$$

$$X = MAX(x[R], x[Gr], x[Gb], x[B]) \quad (4)$$

Herein, MAX() represents a function that returns the maximum of arguments.

The reason the maximum of potential black balance adjustment values determined for each color component is determined as the black balance adjustment value is that the black balance on the observation image is prevented from being insufficiently adjusted by the adjustment unit 33.

However, in a case where the determined black balance adjustment value exceeds an upper limit adjustment value, the adjustment value determination unit 32 determines the upper limit adjustment value as the black balance adjustment value. In other words, in this case, the black balance adjustment value is clipped to the upper limit adjustment value. Specifically, the final black balance adjustment value Eval is determined by the following Formula (5).

$$Eval = MIN(EvalMax, X) \quad (5)$$

Herein, EvalMax represents the upper limit adjustment value, and MIN() represents a function that returns the minimum of arguments.

The reason the black balance adjustment value is clipped to the upper limit adjustment value in a case where the black balance adjustment value exceeds the upper limit adjustment value is that the black balance on the observation image is prevented from being excessively adjusted by the adjustment unit 33.

Note that the threshold Thr and the upper limit adjustment value may be fixed values determined in consideration of the appearance of an observation image after the black balance adjustment or may be dynamically determined depending on a ratio of a sample and a background of an observation image or depending on observation methods which are switchable.

The adjustment unit 33 adjusts the black balance of the observation image generated by the image capturing device 20. Specifically, the adjustment unit 33 subtracts the black balance adjustment value determined by the adjustment value determination unit 32 from the pixel values of every color component in each pixel of the observation image, thereby generating an adjusted observation image. Specifically, the adjusted observation image is generated in the follow manner.

For example, when the observation image is an RGB color image and pixel values of every color component in each pixel of the observation image are R(x, y), G(x, y), and B(x, y), pixel values R'(x, y), G'(x, y), and B'(x, y) of every color component in each pixel of the adjusted observation image are calculated by the following Formula (6).

$$R'(x,y) = R(x,y) - Eval$$
$$G'(x,y) = G(x,y) - Eval$$
$$B'(x,y) = B(x,y) - Eval \quad (6)$$

Note that Eval is the black balance adjustment value determined by the adjustment value determination unit 32.

In a case where the pixel values do not take negative values, the following Formula (7) is used instead of Formula (6), and in addition, the negative pixel values are clipped to 0.

$$R'(x,y) = MAX(0, R(x,y) - Eval)$$
$$G'(x,y) = MAX(0, G(x,y) - Eval)$$
$$B'(x,y) = MAX(0, B(x,y) - Eval) \quad (7)$$

The example using an RGB color image as the observation image has been described above, but an adjusted observation image can be generated similarly in a case where the observation image is a Bayer pattern image. In this case, a black balance adjustment value Eval is subtracted from pixel values of every color component in each pixel (pixel values of R pixel, pixel values of Gr pixel, pixel values of Gb pixel, and pixel values of B pixel) of the Bayer pattern image (in a case where the pixel values do not take negative values, the negative pixel values are clipped to 0), thereby generating an adjusted observation image.

The output device 40 outputs the adjusted observation image generated by the adjustment unit 33. The output device 40 is a display device such as a liquid crystal display or a printer. For example, the output device 40 which is a display device displays the adjusted observation image generated by the adjustment unit 33.

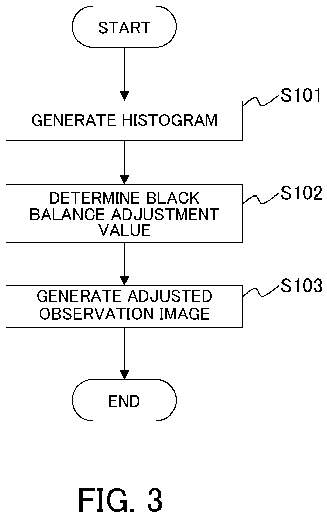

FIG. 3 is a flowchart illustrating image processing performed by the image processing device.

In the image processing illustrated in FIG. 3, when an observation image is input from the image capturing device 20 to the image processing device 30, the histogram generation unit 31 generates a histogram plotting pixel values of every color component in the input observation image (Step S101). Note that the input observation image is generated by the image capturing device 20 that captures an optical observation image (for example, an optical observation image at the time of fluorescence observation) formed by the microscope 10. Details on how to generate the histogram by the histogram generation unit 31 are as described above.

Next, the adjustment value determination unit 32 determines a black balance adjustment value based on the histogram plotting pixel values of every color component generated in Step S101 (Step S102). Details on how to determine the black balance adjustment value by the adjustment value determination unit 32 are as described above.

Next, the adjustment unit 33 subtracts the black balance adjustment value determined in Step S102 from the pixel values of every color component in each pixel of the observation image input, thereby generating an adjusted observation image (Step S103). Details on how to generate the adjusted observation image by the adjustment unit 33 are as described above.

After that, the adjusted observation image generated by the adjustment unit 33 is output from the image processing device 30 to the output device 40 and is displayed by the output device 40 which is, for example, a display device.

In this manner, according to the present embodiment, black balance can be adjusted without calculation of brightness information (calculation according to Formula (1)), whereby the black balance is preferably adjusted without the influence of color components in an observation image. In addition, such black balance adjustment sufficiently subtracts brightness of a background (brightness due to autofluorescence of an optical member) from the observation image at the time of fluorescence observation. Accordingly, it is possible to sufficiently remedy the reduction of contrast between a sample and the background of the observation image.

The present embodiment can also be configured as the following modifications. In the following modifications, the same elements are denoted by the same reference numerals, and details thereof will be omitted.

<First Modification>

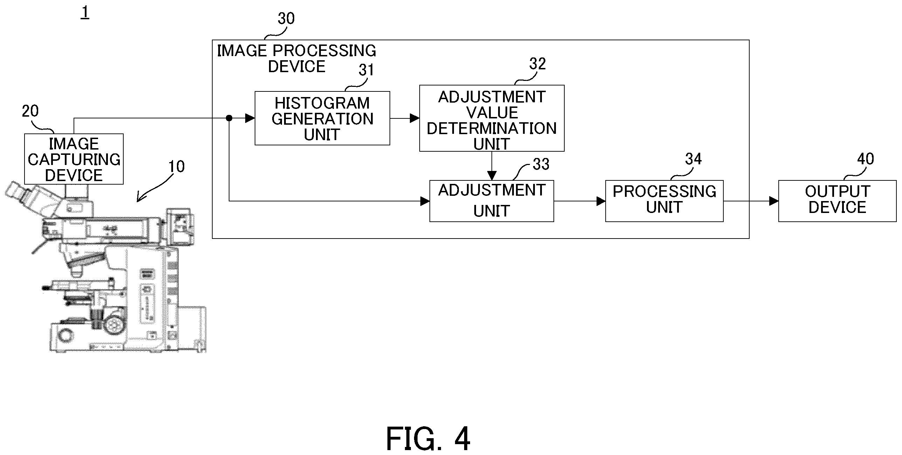

FIG. 4 is a view illustrating a configuration of the microscope system according to a first modification. As compared with the microscope system 1 illustrated in FIG. 1, the microscope system 1 according to the first modification illustrated in FIG. 4 has the image processing device 30 further including a processing unit 34.

In the microscope system 1 according to the first modification, assume that an observation image generated by the image capturing device 20 is a Bayer pattern image, and an adjusted observation image generated by the adjustment unit 33 is also a Bayer pattern image.

The processing unit 34 performs processing of the adjusted observation image generated by the adjustment unit 33, involving conversion of the Bayer pattern image into a color image. The processing involves, for example, de-mosaicing, color operation, gamma correction, edge enhancement, and noise reduction.

Furthermore, in the microscope system 1 according to the first modification, the output device (for example, a display device) 40 outputs (for example, displays) the adjusted observation image processed by the processing unit 34.

According to the first modification, black balance is adjusted before the processing, that is to say, the processing is performed on the good observation image subjected to the black balance adjustment.

<Second Modification>

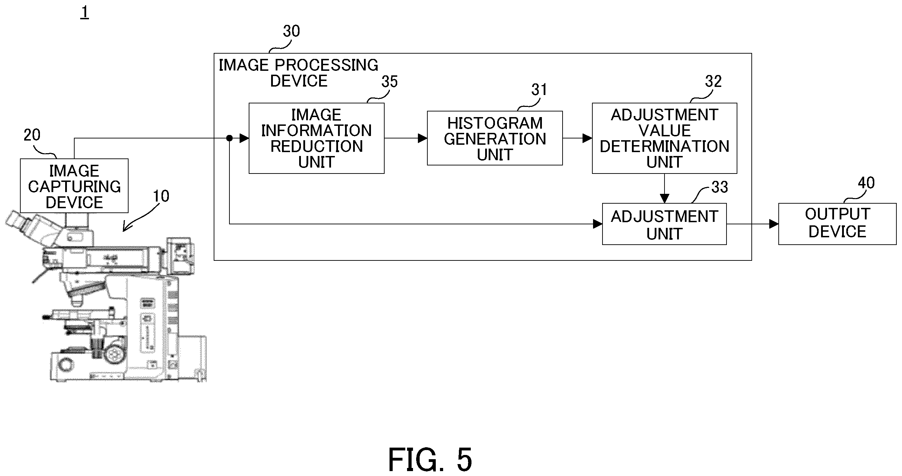

FIG. 5 is a view illustrating a configuration of the microscope system according to a second modification. As compared with the microscope system 1 illustrated in FIG. 1, the microscope system 1 according to the second modification illustrated in FIG. 5 has the image processing device 30 further including an image information reduction unit 35.

The image information reduction unit 35 reduces an amount of information on an observation image generated by the image capturing device 20. The image information reduction unit 35 scales down the observation image by known interpolation such as nearest neighbor interpolation and bilinear interpolation, thereby reducing an amount of information. The image information reduction unit 35 may perform anti-aliasing on the observation image by low pass filtering (LPF) before the interpolation. Accordingly, it is possible to suppress aliasing that may occur on the observation image scaled down by the interpolation. Alternatively, the image information reduction unit 35 may reduce an amount of information by dividing the observation image into predetermined block sizes and setting an average of pixel value of every color component in each pixel included in each block size as a pixel value of every color component in one pixel.

According to the second modification, the histogram generation unit 31 and the adjustment value determination unit 32 process the observation image having the amount of information reduced by the image information reduction unit 35, which speeds up the operations of the histogram generation unit 31 and the adjustment value determination unit 32. Although the image information reduction unit 35 scales down the observation image and reduces its amount of information, it should be noted that such processing has a small impact on the shape of a histogram generated by the histogram generation unit 31 and exerts little influence on accuracy of a black balance adjustment value determined by the adjustment value determination unit 32.

The second modification is particularly effective in a case where an observation image has a large size and a histogram is generated using parallel processing hardware. Examples of the parallel processing hardware include a digital signal processor (DSP) and a single instruction multiple data (SIMD) of a central processing unit (CPU). Typically, the generation of a histogram is not suitable for parallel processing. For this reason, using parallel processing hardware to generate a histogram for a large-size observation image may increase the processing time and make it difficult to enable real-time processing. To solve this problem, in the second modification, a histogram is generated for an observation image having a size and amount of information reduced, which does not increase the processing time and enables real-time processing without difficulty.

<Third Modification>

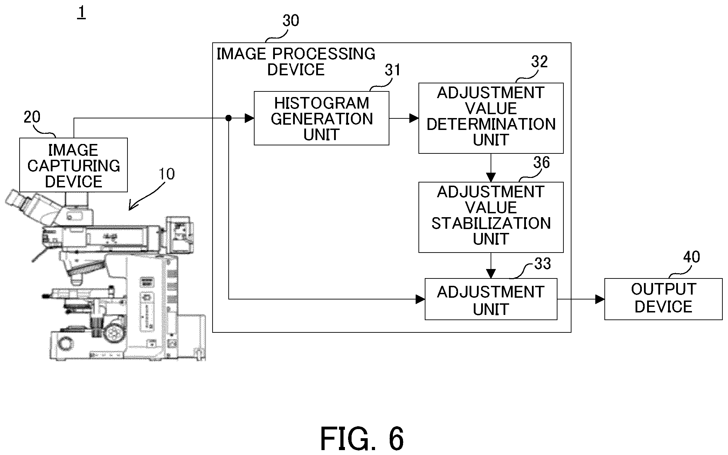

FIG. 6 is a view illustrating a configuration of the microscope system according to a third modification. As compared with the microscope system 1 illustrated in FIG. 1, the microscope system 1 according to the third modification illustrated in FIG. 6 has the image processing device 30 further including an adjustment value stabilization unit 36.

In the microscope system 1 according to the third modification, the image capturing device 20 captures an image (generates an observation image) at a predetermined frame rate, and the image processing device 30 generates an adjusted observation image of the observation image for each frame, and the output device 40 as a display device displays the adjusted observation image.

The adjustment value stabilization unit 36 smooths a black balance adjustment value determined by the adjustment value determination unit 32 and stabilizes the black balance adjustment value in the direction of time. The adjustment value stabilization unit 36 smooths the black balance adjustment value by a moving average filter such as finite impulse response (FIR) filter or infinite impulse response (IIR) filter.

Furthermore, in the microscope system 1 according to the third modification, the adjustment unit 33 generates an adjusted observation image using the black balance adjustment value smoothed by the adjustment value stabilization unit 36.

According to the third modification, for example, even when the black balance adjustment value determined by the adjustment value determination unit 32 varies due to influences of noise at the time of image capturing by the image capturing device 20, smoothing by the adjustment value stabilization unit 36 stabilizes the black balance adjustment value. Accordingly, in the adjusted observation image generated by the adjustment unit 33 and displayed by the output device 40, it is possible to suppress the variation in brightness attributed to the variation in black balance adjustment value, thereby preventing poor visibility of the adjusted observation image which could be caused by the variation in brightness.

In the third modification, in a case where a black balance adjustment value Eval determined by the adjustment value determination unit 32 satisfies the following Formula (8) with respect to a smoothed black balance adjustment value Eval', the adjustment value stabilization unit 36 may update the smoothed black balance adjustment value Eval', assuming that Eval' = Eval.

$$Eval < Eval' - h \text{ or } Eval' + h < Eval \quad (8)$$

The symbol h may be a fixed value or a value dynamically determined depending on a value of Eval'.

An example of the case where the black balance adjustment value Eval satisfies Formula (8) includes a change in observation area at the time of observation, which changes the content of the observation image. Even in such a case, the subsequent black balance adjustment can be performed appropriately.

The first to third modifications have been described so far, but two or more of the first to third modifications may be combined and employed as another modification of the present embodiment.

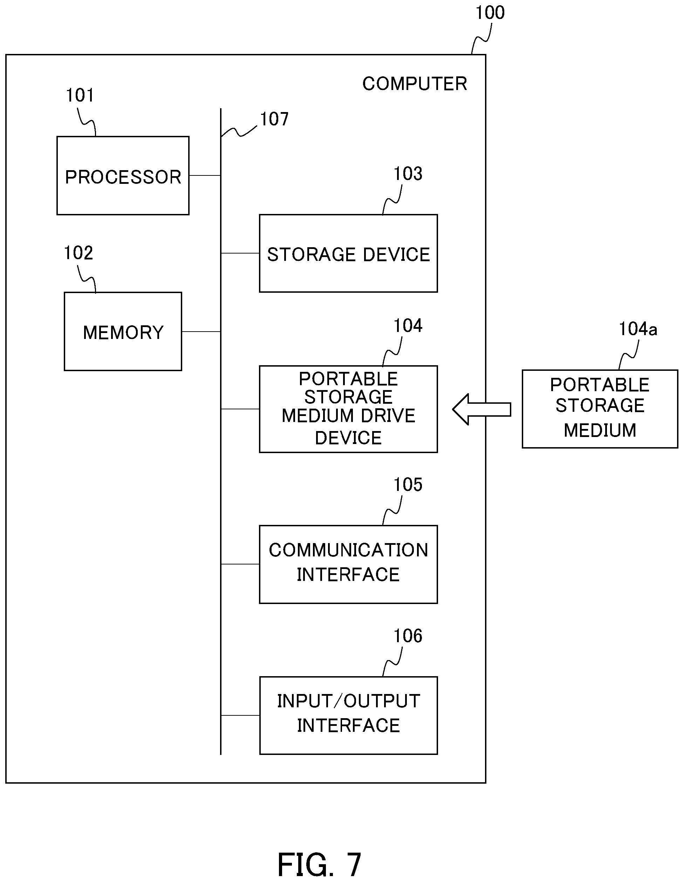

In the microscope system 1, the image processing device 30 may be achieved by a computer 100 illustrated in FIG. 7. FIG. 7 is a view illustrating a hardware configuration of the computer 100.

The computer 100 illustrated in FIG. 7 includes a processor 101, a memory 102, a storage device 103, a portable storage medium drive device 104, a communication interface 105, and an input/output interface 106, and these devices are connected to a bus 107 to transmit and receive data reciprocally.

The processor 101 may be, for example, a single processor, a multiprocessor, or a multi-core processor. The processor 101 performs various types of processing (for example, image processing illustrated in FIG. 3) by executing a program such as an operating system (OS) and an application.

The memory 102 includes a random access memory (RAM) and a read only memory (ROM). A part of the program or the like executed by the processor 101 is temporarily stored in the RAM. The RAM is also used as a working storage area of the processor 101. The ROM stores the program executed by the processor 101 and various types of data necessary for executing the program.

The storage device 103 stores data, and examples of the device include a hard disk drive (HDD) and a solid state drive (SSD).

The portable storage medium drive device 104 drives a portable storage medium 104a, accesses the content stored therein, and reads and writes data. Examples of the portable storage medium 104a include a memory device, a flexible disk, an optical disk, and a magneto-optical disk. Other examples of the portable storage medium 104a include a compact disc read only memory (CD-ROM), a digital versatile disc (DVD), a Blu-ray disc, a universal serial bus (USB) memory, and an SD card memory.

The communication interface 105 is connected to a network in a wired or wireless manner and communicates with an external device connected to the network.

The input/output interface 106 is connected to an external device (for example, the image capturing device 20, the output device 40, and an input device) and inputs and outputs data to and from the external device. Examples of the input device include a keyboard, a mouse, a joystick, and a touch panel.

In such a computer 100, the program executed by the processor 101 and various types of data necessary for executing the program may be stored not only in the memory 102 but also in the storage device 103 or the portable storage medium 104a. Furthermore, the program executed by the processor 101 and various types of data necessary for executing the program may be stored in the storage device 103 or the portable storage medium 104a via the communication interface 105 from an external device connected to a network.

Still further, the computer 100 is not limited to one illustrated in FIG. 7. The number of some components illustrated in FIG. 7 may be two or more. Alternatively, some of the components may be omitted.

The computer 100 may also include hardware such as a microprocessor, a DSP, an application specific integrated circuit (ASIC), a programmable logic device (PLD), and a field-programmable gate array (FPGA). For example, the processor 101 may be implemented with at least one of the aforementioned hardware.

In a case where the image processing device 30 is achieved by the computer 100 illustrated in FIG. 7, functions of the histogram generation unit 31, the adjustment value determination unit 32, the adjustment unit 33, the processing unit 34, the image information reduction unit 35, and the adjustment value stabilization unit 36 are achieved by the processor 101.

Although embodiments and modifications of the present invention have been described, the present invention is not limited to the embodiments and the modifications, and various improvements and changes can be made without departing from the gist of the present invention.

**Claims**

1. A microscope system, comprising:

   a microscope that forms an optical observation image;
   an image capturing device that captures the optical observation image and generates an observation image;
   a histogram generation unit that generates a histogram plotting pixel values of every color component in the observation image;
   an adjustment value determination unit that determines a black balance adjustment value based on the histogram plotting pixel values of every color component;
   an adjustment unit that subtracts the black balance adjustment value from pixel values of every color component in each pixel of the observation image to generate an adjusted observation image; and
   an output device that outputs the adjusted observation image.

2. The microscope system according to claim 1, wherein the adjustment value determination unit determines, as a potential black balance adjustment value, a pixel value of every color component at which a cumulative pixel count from the darkest part of the histogram plotting pixel values of every color component reaches a threshold.

3. The microscope system according to claim 2, wherein the adjustment value determination unit determines a maximum of the potential black balance adjustment value determined for every color component as the black balance adjustment value.

4. The microscope system according to claim 3, wherein the adjustment value determination unit determines an upper limit adjustment value as the black balance adjustment value in a case where the determined black balance adjustment value exceeds the upper limit adjustment value.

5. The microscope system according to any one of claims 1 to 4, further comprising a processing unit that performs processing on the adjusted observation image, the processing involving conversion of a Bayer pattern image into a color image, wherein the output device outputs the adjusted observation image subjected to the processing by the processing unit.

6. The microscope system according to any one of claims 1 to 4, further comprising an image information reduction unit that reduces an amount of information on the observation image,

wherein the histogram generation unit generates a histogram plotting pixel values of every color component in the observation image having an amount of information reduced by the image information reduction unit.

7. The microscope system according to any one of claims 1 to 4, further comprising an adjustment value stabilization unit that smooths the black balance adjustment value,

wherein the adjustment unit generates the adjusted observation image by subtracting the black balance adjustment value smoothed by the adjustment value stabilization unit from pixel values of every color component in each pixel of the observation image.

8. A method of image processing executed by a computer, the method comprising:

generating a histogram plotting pixel values of every color component in an observation image which is generated by an image capturing device capturing an optical observation image formed by a microscope;
determining a black balance adjustment value based on the histogram plotting pixel values of every color component; and
subtracting the black balance adjustment value from pixel values of every color component in each pixel of the observation image to generate an adjusted observation image.

9. A program for causing a computer to implement:

generating a histogram plotting pixel values of every color component in an observation image which is generated by an image capturing device capturing an optical observation image formed by a microscope;
determining a black balance adjustment value based on the histogram plotting pixel values of every color component; and
subtracting the black balance adjustment value from pixel values of every color component in each pixel of the observation image to generate an adjusted observation image.

FIG. 1

EP 4 521 337 A1

FIG. 2

```
          ┌──────────┐
          │  START   │
          └────┬─────┘
               │
               ▼
     ┌──────────────────────┐
     │ GENERATE HISTOGRAM   │──── S101
     └──────────┬───────────┘
                │
                ▼
     ┌──────────────────────┐
     │   DETERMINE BLACK     │──── S102
     │ BALANCE ADJUSTMENT    │
     │       VALUE           │
     └──────────┬───────────┘
                │
                ▼
     ┌──────────────────────┐
     │  GENERATE ADJUSTED    │──── S103
     │ OBSERVATION IMAGE     │
     └──────────┬───────────┘
                │
                ▼
          ┌──────────┐
          │   END    │
          └──────────┘
```

# FIG. 3

1

30

IMAGE PROCESSING DEVICE

31

HISTOGRAM GENERATION UNIT

32

ADJUSTMENT VALUE DETERMINATION UNIT

20

IMAGE CAPTURING DEVICE

10

33

ADJUSTMENT UNIT

34

PROCESSING UNIT

40

OUTPUT DEVICE

FIG. 4

12

FIG. 5

EP 4 521 337 A1

1

30

IMAGE PROCESSING
DEVICE

20

IMAGE
CAPTURING
DEVICE

10

31

HISTOGRAM
GENERATION
UNIT

32

ADJUSTMENT
VALUE
DETERMINATION
UNIT

36

ADJUSTMENT
VALUE
STABILIZATION
UNIT

33

ADJUSTMENT
UNIT

40

OUTPUT
DEVICE

FIG. 6

FIG. 7

Europäisches
Patentamt

European
Patent Office

Office européen
des brevets

# EUROPEAN SEARCH REPORT

Application Number

EP 24 19 8548

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 4 504644 B2 (OLYMPUS CORP) 14 July 2010 (2010-07-14) * the whole document * ----- | 1-9 | INV. G06T5/40 G06T5/92 |
| X | JP 6 184305 B2 (OLYMPUS CORP) 23 August 2017 (2017-08-23) * paragraph [0013] - paragraph [0021] * ----- | 1,8,9 | |
| X | JP 2010 098719 A (OLYMPUS CORP) 30 April 2010 (2010-04-30) * paragraph [0022] - paragraph [0079] * ----- | 1,8,9 | |

TECHNICAL FIELDS
SEARCHED (IPC)

G06T
H04N

The present search report has been drawn up for all claims

| Place of search | Date of completion of the search | Examiner |
|---|---|---|
| Munich | 22 January 2025 | Yilmaz, Özgün |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 19 8548

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2025

| Patent document cited in search report | Publication date | Patent family member(s) | Publication date |
|---|---|---|---|
| JP 4504644 B2 | 14-07-2010 | JP 4504644 B2<br>JP 2005070537 A | 14-07-2010<br>17-03-2005 |
| JP 6184305 B2 | 23-08-2017 | JP 6184305 B2<br>JP 2015105964 A<br>US 2015145982 A1 | 23-08-2017<br>08-06-2015<br>28-05-2015 |
| JP 2010098719 A | 30-04-2010 | JP 5355259 B2<br>JP 2010098719 A | 27-11-2013<br>30-04-2010 |

EPO FORM P0459

For more details about this annex : see Official Journal of the European Patent Office, No. 12/82

**EP 4 521 337 A1**

**Patent documents cited in the description**

- JP 2010098719 A